# EUROPEAN PATENT APPLICATION

(11) **EP 4 104 844 A1**
(43) Date of publication of application: **21.12.2022**
(21) Application number: 20898339.5
(22) Date of filing: 11.12.2020
(51) Int. Cl.: A61K 35/747, A23C 9/123, A23L 33/135, A61P 9/00, A61P 43/00, C12N 1/20

(54) **COMPOSITION FOR LOWERING HEART RATE**

(30) Priority: 13.12.2019 JP 2019225685
(71) Applicant: MEIJI CO., LTD, Chuo-ku Tokyo 104-8306 (JP)
(72) Inventor: TOSHIMITSU Takayuki, Hachioji-shi, Tokyo 192-0919 (JP); SASHIHARA Toshihiro, Hachioji-shi, Tokyo 192-0919 (JP); ASAMI Yukio, Hachioji-shi, Tokyo 192-0919 (JP)
(74) Representative: TBK
(86) International application number: PCT/JP2020/046227
(87) International publication number: WO 2021/117857

(57) **Abstract**

Provided is a composition for reducing heart rate, including a lactic acid bacterium belonging to the genus Lactobacillus. A subject is allowed to ingest a composition containing a lactic acid bacterium belonging to the genus Lactobacillus to reduce heart rate of the subject.

## Description

### CROSS-REFERENCE TO RELATED APPLICATIONS

The present patent application claims a priority based on Japanese Patent Application No. 2019-225685 filed on December 13, 2019, which is incorporated herein by reference in its entirety.

### BACKGROUND OF THE INVENTION

### Field of the Invention

The present invention relates to a composition for reducing heart rate.

### Background Art

It is known that the resting heart rate (pulse rate) sensitively reflects physical or mental stress. Also, it is reported that higher resting heart rate tends to lead higher mortality attributable to cardiovascular and coronary diseases, as well as higher total mortality (e.g., Non-Patent Document 1). In addition, it is known that a resting heart rate of a healthy subject is approximately 80 beats per minute (bpm), and a heart rate above 80 bpm increases cardiovascular complications, morbidity, and mortality. It is also known that a heart rate of 80 to 85 bpm or more should be considered as a criterion for tachyarrhythmia (tachycardia). Therefore, it is reasonable to set the criteria for tachycardia at 80 bpm or more, and in a subject having such a heart rate, reducing the resting heart rate may reduce the risk of cardiovascular and coronary disease, and may also reduce mortality.

It is also known that heart rate is closely related to blood pressure, and that an elevation in heart rate increases the risk of developing diseases such as arteriosclerosis and hypertensive complications (e.g., myocardial infarction and cerebral infarction), for example.

It is conventionally known that certain lactic acid bacteria affect various physiological functions, for example, improve the metabolism of sugars and lipids in mice (e.g., Patent Document 1, Non-Patent Documents 2 and 3).

However, it has not been known that ingestion of lactic acid bacteria can reduce heart rate of the subject to ingest. Since high resting heart rate can increase the risk of cardiovascular and coronary diseases and consequently contribute to high total mortality, there is a need for effective methods to reduce heart rate.

### Prior Art Document

### Patent Document

Patent Document 1: WO2012/014971

### Non-Patent Document

Non-Patent Document 1: HEART's Selection (Thinking about Heart Rate) "Learning from Epidemiology: Heart Rate and Cardiovascular Disease", 2011, Vol. 43, Issue 11, pp. 1397-1401;
Non-Patent Document 2: T. Toshimitsu et al. "Identification of Lactobacillus plantarum strain that meliorates chronic inflammation and metabolic disorders in obese and type 2 diabetic mice" J Dairy Sci, 99:933-946, 2016;
Non-Patent Document 3: Tohru Sakai et al. "Lactobacillus plantarum OLL2712 regulates glucose metabolism in C57BL/6 mice fed a high-fat diet" J Nutr Sci Vitaminol, 59, 144-147, 2013.

### SUMMARY OF THE INVENTION

An object of the present invention is to provide a composition for effectively reducing heart rate in a subject.

As a result of intensive studies, the present inventors have found that lactic acid bacteria belonging to the genus Lactobacillus can reduce heart rate of a subject when the subject is made to ingest the lactic acid bacteria. The present invention is based on these findings.

According to the present invention, the following inventions are provided.
[1] A composition for reducing heart rate, including a lactic acid bacterium belonging to the genus Lactobacillus.
[2] The composition according to [1], which is for human.
[3] The composition according to [1] or [2], wherein the lactic acid bacterium is a lactic acid bacterium having an activity to decrease an amount of Interleukin-6 in the blood.
[4] The composition according to any one of [1] to [3], wherein the lactic acid bacterium can induce production of Interleukin-10 in a mammalian cell.
[5] The composition according to [4], wherein the mammalian cell is selected from a bone marrow-derived dendritic cell and a peritoneal macrophage.
[6] The composition according to any one of [1] to [5], wherein the lactic acid bacterium has a 16S rRNA gene having 90% or more homology with the nucleotide sequence represented by SEQ ID NO: 1.
[7] The composition according to any one of [1] to [6], wherein the lactic acid bacterium is Lactobacillus plantarum.
[8] The composition according to any one of [1] to [7], wherein the lactic acid bacterium is Lactobacillus plantarum OLL2712 strain deposited under accession number FERM BP-11262.
[9] The composition according to any one of [1] to [8], wherein the lactic acid bacterium contains a killed bacterial cell of a lactic acid bacterium.
[10] The composition according to any one of [1] to [9], wherein the lactic acid bacterium contains a heat-killed bacterial cell of a lactic acid bacterium.
[11] The composition according to any one of [1] to [10], wherein the composition is a food composition.
[12] The composition according to any one of [1] to [10], wherein the composition is a pharmaceutical composition.
[13] A method for reducing heart rate of a subject, including making a subject ingest a composition containing a lactic acid bacterium belonging to the genus Lactobacillus.
[14] Use of a lactic acid bacterium belonging to the genus Lactobacillus to reduce heart rate or to produce a composition for reducing heart rate.
[15] A lactic acid bacterium belonging to the genus Lactobacillus for reducing heart rate.

According to the present invention, heart rate can be effectively reduced in a subject including human.

### BRIEF DESCRIPTION OF THE DRAWINGS

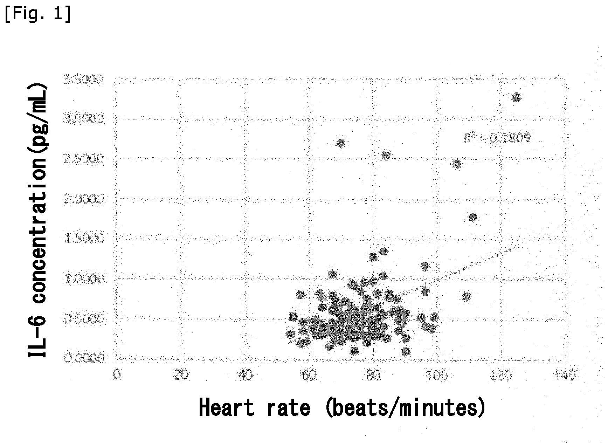

FIG. 1 shows a correlation between an amount of IL-6 in the blood and heart rate in all subjects in the test group and the control group before the start of ingestion of each composition (week 0).

### DETAILED DESCRIPTION OF THE INVENTION

### Composition for Reducing Heart Rate

According to an embodiment of the present invention, there is provided a composition for reducing heart rate of a subject, the composition containing a lactic acid bacterium belonging to the genus Lactobacillus (hereinafter also referred to simply as a "lactic acid bacterium").

According to an embodiment, examples of a subject to whom the composition of the present invention is applied are not particularly limited so long as the effects of the present invention are exhibited, and include a mammal such as a human, a monkey, a chimpanzee, a cow, a horse, a sheep, and a goat, preferably a human. The applicable subject may be a subject having normal heart rate or a subject having heart rate higher than normal heart rate (tachyarrhythmia) (i.e., a tachycardic subject).

The term "normal heart rate" used herein means heart rate at rest in a subject who does not suffer from a disease (e.g., a respiratory disease, a heart disease, a psychiatric disease, fever, etc.), and is at a low risk of death, which may vary depending on the target species, age, gender, etc. On the other hand, the subject having heart rate higher than the normal heart rate range can be said to have tachycardia. For example, normal heart rate in human is 50 to 80 bpm, and heart rate exceeding this range can be said to be tachycardia.

In this specification, "reducing" heart rate of a subject means that, when a lactic acid bacterium or a composition containing a lactic acid bacterium is ingested by or administered to a subject, heart rate is statistically significantly reduced (i.e., beyond the range of error) or heart rate of the subject with a tendency to have an elevated heart rate is suppressed from increasing and maintained at statistically significantly low level, in the subject who is made to ingest the composition or to whom the composition is administered, compared to a subject who is not made to ingest the composition or to whom the composition is administered. For statistical analysis of heart rate, statistical analysis methods known to those skilled in the art can be used. Comparison between groups can be performed using Mann-Whitney U test, whereas comparison within the same group (e.g., comparison of compositions within the same group before and after ingestion) can be performed using Wilcoxon signed-rank sum test. Specifically, the presence or absence of the effect to reduce heart rate reducing effect is determined by the method described in Examples.

The composition of the present invention exhibits a remarkable heart rate reducing effect on subjects with hypertension or tachycardia such as tachyarrhythmia, and on subjects with a tendency to have an elevated heart rate. The composition of the invention reduces heart rate by, for example, 0.5 to 35%, preferably 0.5 to 20%, more preferably 0.5 to 15%, still more preferably 1 to 10%, and even more preferably 1 to 5%, compared to the heart rate without applying the composition of the present invention. Further, the composition of the present invention also exhibits a heart rate reducing effect on a subject having a normal heart rate, the heart rate reducing effect being a mild effect not causing a rapid decrease or an abnormal decrease such as bradycardia. For example, the composition reduces the heart rate in the range of 0.5 to 12%, preferably 1 to 10%, and more preferably 1 to 4%. The composition of the present invention can reduce or maintain heart rate of a subject so that the heart rate is maintained in a normal range. Therefore, the composition of the present invention can be used to improve symptoms caused by cardiovascular diseases (e.g., heart failure, myocardial infarction, angina, arteriosclerosis etc.), coronary disease, hypertension complication (hypertensive cardiac hypertrophy, congestive heart failure, cerebral hemorrhage, cerebral infarction, hypertensive nephropathy), and the like. According to a preferred embodiment of the present invention, improvement of symptoms caused by cardiovascular diseases includes suppression of elevation in heart rate or reduction in heart rate. It should be noted that the term "improvement" used herein encompasses treatment and prevention. The term "treatment" includes not only stopping, alleviating, or delaying the progression or worsening of an abnormality or disease by medical intervention, but also stopping, alleviating, or delaying the progression or worsening of an abnormality or disease by non-medical intervention. The term "prevention" includes preparing in advance for an anticipated worsening of an abnormality or disease and preventing occurrence or recurrence of the abnormality or disease through non-medical or medical intervention.

According to an embodiment of the present invention, the lactic acid bacterium belonging to the genus Lactobacillus contained in the composition of the present invention is preferably a lactic acid bacterium having an activity to decrease the amount of Interleukin-6 (IL-6) in the blood. It is a surprising fact to those skilled in the art that a lactic acid bacterium having an activity to decrease the amount of IL-6 in the blood can be advantageously used for reducing heart rate.

In this specification, "decreasing" IL-6 level means that, when a lactic acid bacterium or a composition containing a lactic acid bacterium is ingested by or administered to a subject, an amount of IL-6 in the subject is statistically significantly decreased (i.e., beyond the range of error), or the amount of IL-6 of the subject with a tendency to have an increased amount of IL-6 is suppressed from increasing and maintained at statistically significantly low level or a statistically significant increase in the amount of IL-6 level is suppressed, in a subject who is made to ingest the composition or to whom the composition is administered, compared to a subject who is not made to ingest the composition or to whom the composition is not administered. Therefore, according to a preferred embodiment, the "activity to decrease the amount of IL-6 in the blood" is an "activity to suppress an increase in the amount of IL-6 in the blood." For statistical analysis of the amount of IL-6 in the blood, statistical analysis methods known to those skilled in the art can be used. Comparison between groups can be performed using Mann-Whitney U test, whereas comparison within the same group (e.g., comparison of compositions within the same group before and after ingestion) can be performed using Wilcoxon signed-rank sum test. Specifically, the presence or absence of the effect to decrease the amount of IL-6 in the blood is determined by the method described in Examples.

According to an embodiment of the present invention, the lactic acid bacterium belonging to the genus Lactobacillus is preferably a lactic acid bacterium capable of inducing production of Interleukin-10 (IL-10) in a mammalian cell. Use of the lactic acid bacterium capable of inducing production of IL-10 is advantageous in reducing heart rate and suppressing inflammation in a mammal that ingests the bacterium. The above-described mammalian cell is preferably selected from a bone marrow-derived dendritic cell and a peritoneal macrophage. The activity of lactic acid bacteria belonging to the genus Lactobacillus to induce IL-10 production can be confirmed by the method described in Examples.

As a lactic acid bacterium contained in the composition of the present invention, any lactic acid bacterium belonging to the genus Lactobacillus can be used without particular limitation. Examples of the lactic acid bacterium belonging to the genus Lactobacillus include Lactobacillus delbrueckii subsp. burgaricus, Lactobacillus delbrueckii subsp. lactis, Lactobacillus casei, Lactobacillus helveticus, Lactobacillus acidophilus, Lactobacillus crispatus, Lactobacillus amylovorus, Lactobacillus gallinarum, Lactobacillus gasseri, Lactobacillus oris, Lactobacillus rhamnosus, Lactobacillus johnsonii, Lactobacillus fermentum, Lactobacillus brevis, Lactobacillus plantarum, Lactobacillus pentosus, Lactobacillus paraplantarum, Lactobacillus paracollinoides, Lactobacillus hammesii. As the lactic acid bacterium, Lactobacillus plantarum is preferably used, and Lactobacillus plantarum OLL2712 strain is more preferably used.

The Lactobacillus plantarum OLL2712 strain is deposited at the Patent organism Depositary in the National Institute of Advanced Industrial Science and Technology (Central 6, 1-1-1 Higashi, Tsukuba, Ibaraki, Japan) on July 2, 2010, then transferred to the International Depositary under accession number FERM BP-11262. As described in Budapest Notification No. 282 (http://www.wipo.int/treaties/en/notifications/budapest/treaty_ budapest_282.html), the National Institute of Technology and Evaluation (IPOD, NITE) has taken over the patent microorganism deposition services from the International Patent Organism Depositary of the National Institute of Advanced Industrial Science and Technology (IPOD, AIST), and therefore the Lactobacillus plantarum OLL2712 strain is now deposited at National Institute of Technology and Evaluation (IPOD, NITE) (#120, 2-5-8 Kazusakamatari, Kisarazu-shi, Chiba, Japan) under accession number FERM BP-11262.

As the lactic acid bacterium contained in the composition of the present invention, a strain substantially equivalent to the above-described deposited strain can also be used. The substantially equivalent strain refers to, for example, a strain of the above-described lactic acid bacterium belonging to the genus Lactobacillus, the strain having the 16S rRNA gene whose nucleotide sequence shows 90% or more, preferably 95% or more, more preferably 98% or more, and even more preferably 99% or more homology to the nucleotide sequence (SEQ ID NO: 1) of the 16S rRNA gene of the above-described deposited strain, and preferably having the mycological properties identical to those of the above-described strain. A strain having the same mycological properties is preferably a strain having an activity similar to that of the above-described deposited strain in that it has an activity to decrease the amount of IL-6 in the blood in a subject (e.g., human). A strain having the same mycological properties is preferably a strain having an activity similar to that of the above-described deposited strain in that it has an activity to induce production of IL-10, preferably in a mammalian cell, more preferably in a mammalian bone marrow-derived dendritic cell or peritoneal macrophage. Furthermore, the lactic acid bacterium contained in the composition of the present invention may be a strain bred from a deposited strain or a substantially equivalent strain by mutation treatment, genetic recombination, selection of a natural mutant, etc., as long as the effects of the present invention are exhibited.

Examples of the lactic acid bacterium contained in the composition of the present invention include, in addition to a lactic acid bacterial cell itself, a culture containing the lactic acid bacterial cell. As the lactic acid bacterial cells, both live bacterial cells and killed bacterial cells can be used. However, killed bacterial cells are preferred, and killed bacterial cells obtained by heat-treating the live bacterial cells (heat-killed bacterial cells) are more preferred. That is, the lactic acid bacterium contained in the composition of the present invention include, preferably killed bacterial cells, more preferably heat-killed bacterial cells. The number of passages of the lactic acid bacterium is not particularly limited as long as the effects of the present invention are exhibited, but is, for example, 1 to 30, preferably 5 to 15, and more preferably 11 to 13.

Examples of a culturing condition for lactic acid bacteria are not particularly limited as long as the effects of the present invention are exhibited, and may include conditions for generally culturing lactic acid bacteria. For example, as a medium, a medium can be used which is obtained by dissolving whey powder and whey protein concentrate in sterile water, digesting with protease A, adding yeast extract, fish extract, and MnSO₄, and further adding various nutrients (vitamins, minerals, fatty acid ester), adjusting pH to 6.7 with NaOH, and then autoclaving for sterilizing. In addition, the pH during culture can be 4.8 to 6.8. K₂CO₃ can be used to adjust pH. Temperature during culturing can be 29 to 40°C.

The heat-treatment for obtaining the heat-killed bacterial cells is not particularly limited as long as the effects of the present invention are exhibited, and is carried out under the conditions usually used for sterilizing lactic acid bacteria.

As the lactic acid bacterium, a bacterium can be used which has been subjected to, in addition to the heat-treatment described above, concentration or dilution, freezing, drying, powdering, or the like.

As the lactic acid bacterium contained in the composition of the present invention, the bacterium prepared by the above-described culture or various treatment, or a commercially available composition containing the lactic acid bacterium may be used.

In the composition of the present invention, the number of the lactic acid bacteria per mass of the composition is not particularly limited as long as the effects of the present invention are exhibited, but is preferably 10⁶ to 10¹⁴ cells/g, more preferably 10⁷ to 10¹³ cells/g, more preferably 10⁸ to 10¹² cells/g, and particularly preferably 10⁸ to 10¹⁰ cells/g. Further, in the composition of the present invention, the dry mass of cells per mass of solid content in the composition is preferably 0.01 to 100% by mass, more preferably 1 to 80% by mass, and even more preferably 10 to 40% by mass.

The composition may contain ingredients other than lactic acid bacteria as long as they do not interfere with the effects of the invention of the present application. Example of the ingredient other than the lactic acid bacterium include medium ingredients, additives suitable for oral ingestion or by tube, solvent such as water, carbohydrates, proteins, lipids, vitamins, minerals, bio-essential trace metals (manganese sulfate, zinc sulfate, magnesium chloride, potassium carbonate, etc.), flavorings, hood sanitarily or pharmaceutically acceptable carriers, and food additives.

Examples of the carbohydrate include sugars, processed starches (dextrin, soluble starch, british starch, oxidized starch, starch esters, starch ethers, etc.), and dietary fiber.

Example of the protein include animal or vegetable proteins such as whole milk powder, skimmed milk powder, partially skimmed milk powder, casein, whey powder, whey protein, whey protein concentrate, whey protein isolate, α-casein, β-casein, κ-casein, β-lactoglobulin, α-lactalbumin, lactoferrin, soybean protein, egg protein, meat protein, hydrolyzed products of these proteins, various milk-derived ingredients such as butter, milky minerals, cream, whey, non-protein nitrogen, sialic acid, phospholipids, and lactose.

Examples of the lipid include animal fats and oils such as lard, fish oil, and their fractionated, hydrogenated, and ester exchange oils; and vegetable fats and oils such as palm oil, safflower oil, corn oil, rapeseed oil, palm oil, and their fractionated, hydrogenated, and ester exchange oils.

Examples of the vitamins include vitamin A, carotene, vitamin B-complex, vitamin C, vitamin D-complex, vitamin E, vitamin K-complex, vitamin P, vitamin Q, niacin, nicotinic acid, pantothenic acid, biotin, inositol, choline, and folic acid.

Examples of minerals include calcium, potassium, magnesium, sodium, copper, iron, manganese, zinc, and selenium.

The composition of the present invention can be produced by blending, in addition to the lactic acid bacterium belonging to the genus Lactobacillus, a pharmaceutically acceptable carrier and/or additives, a food sanitarily acceptable carrier and/or additives and the like, as described above. Therefore, according to another aspect of the present invention, there is provided a method for producing a composition for reducing heart rate, including blending a lactic acid bacterium belonging to the genus Lactobacillus.

According to an embodiment of the present invention, the composition of the present invention for reducing heart rate can be provided as a food composition. The food composition of the present invention may be used for promotion of reduction in heart rate, for suppression of elevation in heart rate, maintenance of normal heart rate, for prevention and/or treatment of diseases such as cardiovascular disease (e.g., heart failure, myocardial infarction, angina, arteriosclerosis etc.), coronary disease, and hypertension complication (hypertensive cardiac hypertrophy, congestive heart failure, cerebral hemorrhage, cerebral infarction, and hypertensive nephropathy).

The food composition of the present invention can be any form of food that can contain the lactic acid bacterium, and can be in any form that can be ingested orally or by tube, such as solution, suspension, emulsion, powder, paste, semi-solid molding, and solid molding. Specific examples of the food include milk, dairy drinks, soft drinks, fermented milk, lactic acid bacteria beverages, lactic beverages, yogurt, cheese, ice cream, frozen sweets, chocolate, tablets (tablet type candy), gummies, candy, bread, biscuits, crackers, pizza crust, formula milk, liquid milk, liquid diet, foods for the sick, nutritional foods, frozen foods, processed foods, seasonings, and other commercially available foods.

The food compositions of the present invention can be food and beverage products labeled for the following uses: for promotion of a reduction in heart rate, for suppression of an elevation in heart rate, for maintenance of a normal heart rate, for promotion of a decrease in risk of cardiovascular disease, for promotion of a decrease in risk of coronary disease, for promotion of a decrease in risk of hypertension complications, for promotion of the production of IL-10, for suppression of the production of IL-6, for activation of the parasympathetic nervous system, and the like. Specifically, the food and beverage products can be labeled "for promotion of a reduction in heart rate," "for suppression of an elevation in heart rate," "for maintenance of a normal heart rate," "for prevention of cardiovascular disease," "prevention of coronary disease," "prevention of hypertension complications," "for promotion of the production of IL-10," "for suppression of the production of IL-6," "for activation of parasympathetic nervous system," "for assisting relaxation", and the like. Other indications can be used as well, as long as they indicate the effect produced by promotion of the production of IL-10 and suppression of the production of IL-6.

In this specification, "indication" means all actions to notify consumers of the above-described use, and any indication that may evoke or analogize the above-described use can be considered as "indication" used in the present invention, regardless of the purpose of the indication, the content of the indication, the object and medium of the indication, etc. However, it is preferable to indicate with expressions that enable consumers to directly recognize the above-mentioned uses.

It is preferable that the indication be an indication that is authorized by the government, etc. (e.g., authorized based on various systems established by the government and indicated in a manner based on such authorization). Examples include labeling as health foods, functional foods, foods with functional claims, enteral foods, foods for special dietary uses, foods for the sick, food with nutrient function claims, quasi-pharmaceutical products, and the like; other indications approved by the Health Labor and Welfare Ministry such as food for specified health use; and indications approved by similar systems. Examples of the latter include labeling as a food for specified health use, labeling as a qualified food for specified health use, labeling for the effect that the product affects the structure and function of the body, and labeling for reducing the risk of disease. More specifically, examples include a labeling as a food for specified health use (especially the labeling for health use) prescribed by Ordinance of the Ministry of Health, Labor and Welfare (April 30, 2003, Ministry of Health, Labor and Welfare Ordinance Issue 86), and similar labeling.

According to another aspect of the present invention, the composition of the present invention for reducing heart rate can be provided as a pharmaceutical composition. The pharmaceutical composition of the present invention may be used for promotion of reduction in heart rate, for suppression of elevation in heart rate, maintenance of normal heart rate, for prevention and/or treatment of diseases such as cardiovascular disease (e.g., heart failure, myocardial infarction, angina, arteriosclerosis etc.), coronary disease, and hypertension complication (hypertensive cardiac hypertrophy, congestive heart failure, cerebral hemorrhage, cerebral infarction, and hypertensive nephropathy). The pharmaceutical composition of the present invention can be produced according to the ordinary producing procedure of the food product except that it contains lactic acid bacteria. The term "pharmaceutical composition" used herein means the composition of the present invention prepared as an oral formulation or a parenteral formulation according to a conventional method. The formulation may be accompanied by additives acceptable for formulation. Examples of additives acceptable for formulation include excipients, stabilizers, preservatives, wetting agents, emulsifiers, lubricants, sweeteners, colorants, flavorings, buffers, antioxidants, and pH adjusters. The pharmaceutical composition as an oral formulation can be in the form of a solid preparation such as tablets, powders, fine granules, granules, capsules, pills, and sustained-release agents, and liquid preparations such as solutions, suspensions and emulsions, or liquid formulations such as solutions, suspensions, and emulsions. The pharmaceutical composition as a parenteral formulation can be in the form of an injection or suppository. From the viewpoint of simplicity of ingestion (administration) to a subject to ingest, the pharmaceutical composition is preferably an oral preparation.

The amount of the composition of the present invention to be ingested is not particularly limited as long as the effects of the present invention are exhibited, and can be appropriately adjusted according to age, health condition, body weight, and the like of the subject to ingest. Typically, it is 0.01 to 10000 mg/kg body weight per day, preferably 0.1 to 1000 mg/kg body weight, more preferably 0.5 to 300 mg/kg body weight, even more preferably 1 to 100 mg/kg body weight. The dry mass of the lactic acid bacteria is preferably 0.001 to 1000 mg/kg body weight, more preferably 0.01 to 100 mg/kg body weight, more preferably 0.05 to 30 mg/kg body weight, and even more preferably 0.1 to 10 mg/kg body weight. The number of lactic acid bacteria is preferably 10⁴ to 10¹² cells/kg body weight, more preferably 10⁵ to 10¹¹ cells/kg body weight, still more preferably 10⁶ to 10¹⁰ cells/kg body weight, and particularly preferably 10⁶ to 10⁸ pieces/kg body weight.

The composition of the present invention is preferably continuously ingested (administered) over a long period of time, specifically, continuously ingested (administered) for 3 days or more, and more preferably continuously ingested (administered) for 1 week or more, in order to exert its effect better. Examples of the ingestion (administration) period include 1 to 6 weeks, 1 to 12 weeks, 2 to 10 weeks, 4 to 10 weeks, and 4 to 12 weeks. As used herein, "continuously" means continuing to ingest a predetermined amount of the composition of the present invention daily.

According to another aspect of the present invention, there is provided a method for reducing heart rate in a subject, including making the subject ingest an effective amount of a lactic acid bacterium belonging to the genus Lactobacillus and/or a composition containing the lactic acid bacterium belonging to the genus Lactobacillus.

Examples of the subject to ingest the lactic acid bacterium and/or composition in the above-described method are not particularly limited as long as they are subjects who require to reduce their heart rate, and include subjects with symptoms of tachyarrhythmia, and subjects with a tendency to have an elevated heart rate.

In the method for reducing heart rate, an amount and period of the ingestion of the above-described lactic acid bacterium and/or composition are not particularly limited as long as the effects of the present invention are exhibited, and can be appropriately adjusted according to age, health condition, body weight, and the like of the subject to ingest. Typically, in the method of the present invention, the amount and period of the ingestion of the above-described lactic acid bacterium and/or composition are similar to the amount and period of the lactic acid bacterium in the composition of the present invention.

According to another aspect of the present invention, there is provided a method for treating and/or preventing the disease and/or disorder caused by elevated heart rate in a subject, including making the subject ingest an effective amount of a lactic acid bacterium belonging to the genus Lactobacillus and/or a composition containing the lactic acid bacterium belonging to the genus Lactobacillus.

Examples of the disease, disorder caused by elevated heart rate include, without particular limitation, cardiovascular disease (e.g., heart failure, myocardial infarction, angina, arteriosclerosis etc.), coronary disease, hypertension complication (hypertensive cardiac hypertrophy, congestive heart failure, and cerebral hemorrhage, cerebral infarction, hypertensive nephropathy).

According to another aspect of the present invention, there is provided the use of the lactic acid bacterium belonging to the genus Lactobacillus for reducing heart rate.

According to another aspect of the present invention, there is provided the use of the lactic acid bacterium belonging to the genus Lactobacillus for producing the composition for reducing heart rate.

According to another aspect of the present invention, there is provided the lactic acid bacterium belonging to the genus Lactobacillus for reducing heart rate.

As the lactic acid bacterium belonging to the genus Lactobacillus used in the above-described another aspect, the lactic acid bacterium belonging to the genus Lactobacillus used in the above- described composition of the present invention can be used.

### EXAMPLES

The present invention will be specifically described with reference to the following examples, but the present invention is not limited to these examples.

### Example 1: Confirmation of heart rate reducing effect of composition containing lactic acid bacterium

### (1) Selection (Screening) of Test Subject

Test subjects were selected for test to confirm the heart rate reducing effect based on the following Selection Criterion A and Exclusion Criterion B.

### A: Selection Criterion

· Healthy men and women between the ages of 20 and 64
· Fasting blood glucose between 100 and 125 mg/dL at screening test
· Blood hemoglobin A1c (HbA1c) level of 5.6 to 6.4 at screening test
· According to the description in "Report of the Committee on the classification and diagnostic criteria of diabetes mellitus (International Standardization Compliant Version)," Seino et al, Diabetology Vol. 55, Issue 7 (2012), Subjects who meet the fasting blood glucose and HbAlc values shown in Selection Criterion A are diagnosed to be included in: a group in which (suspicion of present diabetes mellitus cannot be ruled out, and a group with at high risk of developing diabetes mellitus in the future, even if they do not have it currently. That is, the subjects who meet the selection criterion A are considered to be experiencing a decline in the metabolic function of sugars and lipids due to chronic inflammation of adipose tissue associated with aging and unhealthy lifestyle habit. Specifically, it is known that an increase in inflammatory cytokines such as monocyte chemotactic protein-1 (MCP-1) and IL-6 in adipose tissue increases macrophage infiltration into adipose tissue and decreases the production of hormones necessary for the normal metabolism of sugars and lipids (Xu H et al. "Chronic inflammation in fat plays a crucial role in the development of obesity-related insulin resistance" J Clin Invest, 112: 1821-1830, 2003; and Weisberg SP et al. "CCR2 modulates inflammatory and metabolic effects of high-fat feeding" J Clin Invest, 116: 115-124, 2006). Thus, subjects who meet the fasting blood glucose and HbAlc values shown in selection criterion A are likely to have a tendency for the amount of IL-6 in the blood to increase over time without intervention such as improvement of lifestyle habit. Furthermore, because this study was conducted from August to November, when humans tend to gain weight, the amount of IL-6 produced by adipocytes tended to increase, resulting in an increase in the amount of IL-6 in the blood.

### B: Exclusion Criterion

· Undergoing drug treatment that affects blood glucose level
· Having a habit of ingesting supplements or health foods that affect blood glucose levels within a month before the screening test
· Having a habit of ingesting fermented milk or lactic acid bacteria beverage at least 3 times a week within 3 months before the screening test
· Having an allergy to milk
· Diagnosed with diabetes as a result of screening tests and requiring drug treatment
· Suffering from severe systemic diseases
· Suffering from chronic diseases requiring daily drug treatment
· Judged to be unsuitable as subjects based on clinical examination at the time of screening test
· Received a blood transfusion of more than 200mL within 1 month, or received a blood transfusion of more than 400mL within 3 months
· Heavy alcohol drinkers
· Alcoholics or drug addicts
· Participated in other clinical trial(s) within one month prior to obtaining consent to participate in this study
· Pregnant or breastfeeding
· Judged by the investigator to be inappropriate as subjects.

From 1593 applicants, 126 subjects were selected based on the above criteria. Table 1 shows the age, gender, body weight, and BMI of the subjects in the control group and the test group.

**[Table 1]**

| | control group (n=64) | test group (n=62) |
|---|---|---|
| age (y/o) | 51.2±7.6 | 50.6±6.9 |
| men / women | 44/20 | 42/20 |
| body weight (kg) | 69.4±12.3 | 69.1±11.0 |
| BMI (kg/m²) | 24.9±3.2 | 24.7±3.3 |

| | | |
|---|---|---|
| average ± standard deviation | | |

### (2) Preparation of Composition

Lactobacillus delbrueckii subsp. bulgaricus and Streptococcus thermophilus as lactic acid bacteria starters were added to a mixture containing raw milk, skimmed milk powder, cream, sugar and stevia, and the mixture was fermented for about 3 hours in a temperature environment of 43°C to prepare yogurt. The heat-treated Lactobacillus plantarum OLL2712 strain (accession number: FERM BP-11262) was added to the prepared yogurt to obtain 112 g of the composition (test composition) to be ingested by the test group. The amount of lactic acid bacteria in the test composition was 5 × 10⁹ or more/112 g. On the other hand, 112 g of the same yogurt used in the test composition (yogurt without containing heat-treated Lactobacillus plantarum OLL2712 strain (accession number: FERM BP-11262)) was used as the composition (control composition) to be ingested by the control group. Each composition was stored refrigerated until ingested by subjects in each group. The heat treatment of the lactic acid bacteria was performed by concentrating the bacterial cells to the above concentration and then heating at 60°C for 10 minutes.

### (3) Effect on amount of IL-6 in blood

The effect of the composition of the present invention on the amount of IL-6 in the blood was evaluated according to the following procedure. First, each subject (62 subjects) in the test group selected in (1) described above was made to ingest 112 g of the test composition daily for 12 weeks. In addition, each subject (64 subjects) in the control group selected in (1) described above was made to ingest 112 g of the control composition daily for 12 weeks. For each group, blood samples were taken at 4, 8, and 12 weeks after the start of ingestion, and the amount (concentration) of IL-6 in the blood was measured using serum with the Bio-Plex multiplex system.

Measurements of IL-6 at each time point were evaluated by the Mann-Whitney U test. The results are shown in Table 2. All values in the table are in pg/mL.

**[Table 2]**

| | group | before the start of ingestion week 0 | after the start of ingestion 4 weeks | after the start of ingestion 8 weeks | after the start of ingestion 12 weeks |
|---|---|---|---|---|---|
| IL-6 | control group (n=62) | 0.532±0.268 | 0.496±0.250 | 0.563±0.310 | 0.601±0.265^{∗} |
| | test group (n=64) | 0.801±1.231 | 0.855±1.456 | 0.779±1.453 | 0.792±1.296 |

| | | | | | |
|---|---|---|---|---|---|
| average ± standard deviation ^{∗}P<0.05(compared to before the start of ingestion) | | | | | |

As shown in Table 2, the amount of IL-6 in the blood in the test group made to ingest the test composition was not significantly increased, but slightly decreased after the start of ingestion of the test composition compared to that before the start of ingestion. On the other hand, in the control group made to ingest the control composition, the amount of IL-6 in the blood tended to increase from the start till 12 weeks after the ingestion of the control composition, a significant increase was observed 12 weeks after the start of the ingestion. As described "A: Selection Criterion" in (1), the subject who tend to have an increased amount of IL-6 in the blood is targeted in this example. In the light of that, it was shown that the increase in the amount of IL-6 in the blood was suppressed (i.e., the amount of IL-6 in the blood was reduced) in the test group, while the increase in the amount of IL-6 in the blood was not suppressed (i.e., the amount of IL-6 in the blood was not reduced) in the control group.

### (4) Effect on production of Interleukin-10 in bone marrow-derived dendritic cell and peritoneal macrophage

The effects of Lactobacillus plantarum OLL2712 strain on the production of interleukin-10 (IL-10) in each of the bone marrow-derived dendritic cells and the peritoneal macrophages were evaluated according to the following procedure.

The amount of IL-10 produced by the lactic acid bacteria in the bone marrow-derived dendritic cells was measured as follows. Bone marrow fluid extracted from the femur of BALB/c mice (produced by Japan SLC Co., Ltd.) was passed through a 70 µm cell strainer and then hemolyzed, and rabbit IgG was added to prevent non-specific adsorption. Then, biotin-labelled anti-CD4 antibody, anti-CD8 antibody, and I-A^{d} (MHCII marker) antibody were added and allowed to stand on ice for 30 minutes. Then, streptavidin magnetic beads and anti-B220 antibody magnetic beads were added. After passing through a 40 µm cell strainer again, the negative fraction was collected by auto MACS DEPLETE. By this operation, T cells, B cells and antigen-presenting cells were removed from the cells in the bone marrow fluid, and only immature dendritic cells could be separated.

The obtained immature dendritic cells were cultured in 10 ml of RPMI-10 containing 10% GM-CSF, and after 3 days, 5 ml of RPMI-10 containing GM-CSF was added. After another 5 days, the floating cells were collected and used as bone marrow-derived dendritic cells.

The obtained immature dendritic cells were seeded in a 96-well plate at 10⁵ cells/well, and 10 ug/mL of heat-killed bacterial cells of the lactic acid bacterium Lactobacillus plantarum OLL2712 strain were added. After 24 hours, the culture supernatant was collected and the amount (concentration) of IL-10 in the collected culture supernatant was measured using a mouse ELISA kit to determine the amount of IL-10 produced in the bone marrow-derived dendritic cells. Both antibodies and ELISA kits used were purchased from Becton, Dickinson and Company.

The amount of IL-10 produced in the bone marrow-derived dendritic cells was measured by the above procedure. The amount of IL-10 produced in the bone marrow-derived dendritic cells was 1319 ± 62 pg/mL (average ± standard deviation) when Lactobacillus plantarum OLL2712 strain was added at a concentration of 10 µg/mL. In contrast, the amount of IL-10 produced in the bone marrow-derived dendritic cells without Lactobacillus plantarum OLL2712 strain was 0 pg/mL.

These results show that the Lactobacillus plantarum OLL2712 strain has an activity to induce IL-10 production in a bone marrow-derived dendritic cell.

The amount of IL-10 produced by the lactic acid bacterium in the peritoneal macrophage was measured as follows. Two milliliters of 4% thioglycolate medium (Becton, Dickinson and Company) was intraperitoneally administered to BALB/c mice (produced by Japan SLC, Inc.), and the animals were bred for 4 days. After 5 ml of PBS (washing solution) was intraperitoneally injected, the washing solution was collected to obtain peritoneal macrophages.

The obtained peritoneal macrophages were seeded in a 96-well plate at 10⁵ cell/well, and 10 µg/mL of the heat-killed bacterial cells of the lactic acid bacterium Lactobacillus plantarum OLL2712 strain were added. After 24 hours, the culture supernatant was collected and the amount (concentration) of IL-10 in the collected culture supernatant was measured using a mouse ELISA kit to determine the amount of IL-10 produced in the peritoneal macrophage. Both antibodies and ELISA kits used were purchased from Becton, Dickinson and Company.

The amount of IL-10 produced in the peritoneal macrophage was measured by the above procedure. The amount of IL-10 produced in the peritoneal macrophage was 1178 ± 298 pg/mL (average ± standard deviation) when Lactobacillus plantarum OLL2712 strain was added at a concentration of 10 µg/mL. In contrast, the amount of IL-10 produced in the peritoneal macrophage without Lactobacillus plantarum OLL2712 strain was 0 pg/mL.

These results show that the Lactobacillus plantarum OLL2712 strain has an activity to induce IL-10 production in a peritoneal macrophage.

### (5) Evaluation of heart rate reducing effect

The effect of the composition of the present invention on the heart rate was evaluated according to the following procedure.

First, the heart rate of each subject (62 subjects) in the test group was measured immediately before the start of ingestion of the test composition. Then, each subject in the test group was made to ingest 112 g of the test composition daily for 12 weeks. On the other hand, the heart rate of each subject (64 persons) in the control group was measured immediately before the start of ingestion of the control composition. Then, each subject in the control group was made to ingest 112 g of the control composition daily for 12 weeks. During the study, subjects in each group were asked to maintain a normal diet and lifestyle habit (e.g., quantity and quality of exercise). Twelve weeks after the start of ingestion of each composition, the heart rate of each subject was measured on the upper arm after waiting at rest. The measured heart rate of each subject at each time point was analyzed and evaluated by the Wilcoxon signed-rank sum test. The results are shown in Table 3.

**[Table 3]**

| | control group (n=64) | | test group (n=62) | |
|---|---|---|---|---|
| | before the start of ingestion week 0 | after the start of ingestion 12 weeks | before the start of ingestion week 0 | after the start of ingestion 12 weeks |
| heart rate (beats/ minutes) | 76.4±11.4 | 76.7±12.1 | 76.7±12.2 | 73.8±12.2^{∗} |

| | | | | |
|---|---|---|---|---|
| average ± standard deviation ^{∗}P<0.05(compared to before the start of ingestion) | | | | |

As shown in Table 3, in the test group, the heart rate 12 weeks after the start of ingestion of the test composition was significantly lower than that immediately before the start of ingestion. On the other hand, in the control group, there was no significant change between the heart rate at 12 weeks after the start of ingestion of the control composition and the heart rate immediately before the start of ingestion. The results indicate that the composition of the present invention significantly reduces heart rate in the subject.

### (6) Correlation between amount of IL-6 in the blood and heart rate

The correlation between the amount of IL-6 in the blood and the heart rate was examined according to the following procedure. Specifically, the correlation between the amount of IL-6 in the blood of all subjects in the test and control groups before the start of ingestion of each composition (0 weeks), as measured in (3) above, and the heart rate of all subjects in the test and control groups before the start of ingestion of each composition (0 weeks), as measured in (5) above was confirmed. FIG. 1 shows a correlation between an amount of IL-6 in the blood and heart rate in all subjects in the test group and the control group before the start of ingestion of each composition (week 0).

The results in FIG. 1 shows that the amount of IL-6 in the blood significantly correlated with heart rate (R = 0.425, P<0.001).

As described above, the results in Table 2 shows that in the test group made to ingest the test composition, the amount of IL-6 in the blood tends to be suppressed from increasing, and even tends to decrease. Further, the results in FIG. 1 shows that a high correlation is observed between the amount of IL-6 in the blood and heart rate. These results suggest that heart rate in the test group is reduced due to the suppression of the increase in the amount of IL-6 in the blood in the subject. Industrial availability

The invention can significantly reduce heart rate in a subject and reduce the risk of cardiovascular disease, coronary disease, hypertensive complications, and other diseases caused by elevated heart rate.

## Claims

1. A composition for reducing heart rate, comprising a lactic acid bacterium belonging to the genus Lactobacillus.

2. The composition according to claim 1, which is for human.

3. The composition according to claim 1 or 2, wherein the lactic acid bacterium is a lactic acid bacterium having an activity to decrease an amount of Interleukin-6 in the blood.

4. The composition according to any one of claims 1 to 3, wherein the lactic acid bacterium can induce production of Interleukin-10 in a mammalian cell.

5. The composition according to claim 4, wherein the mammalian cell is selected from a bone marrow-derived dendritic cell and a peritoneal macrophage.

6. The composition according to any one of claims 1 to 5, wherein the lactic acid bacterium has a 16S rRNA gene having 90% or more homology with the nucleotide sequence represented by SEQ ID NO: 1.

7. The composition according to any one of claims 1 to 6, wherein the lactic acid bacterium is Lactobacillus plantarum.

8. The composition according to any one of claims 1 to 7, wherein the lactic acid bacterium is Lactobacillus plantarum OLL2712 strain deposited under accession number FERM BP-11262.

9. The composition according to any one of claims 1 to 8, wherein the lactic acid bacterium contains a killed bacterial cell of a lactic acid bacterium.

10. The composition according to any one of claims 1 to 9, wherein the lactic acid bacterium contains a heat-killed bacterial cell of the lactic acid bacterium.

11. The composition according to any one of claims 1 to 10, wherein the composition is a food composition.

12. The composition according to any one of claims 1 to 10, wherein the composition is a pharmaceutical composition.

13. A method for reducing heart rate of a subject, comprising making a subject ingest a composition containing a lactic acid bacterium belonging to the genus Lactobacillus.

14. Use of a lactic acid bacterium belonging to the genus Lactobacillus to reduce heart rate or to produce a composition for reducing heart rate.

15. A lactic acid bacterium belonging to the genus Lactobacillus for reducing heart rate.
